# EUROPEAN PATENT SPECIFICATION

(11) **EP 0 762 893 B1**
(45) Date of publication and mention of the grant of the patent: **12.09.2001**
(21) Application number: 95920114.6
(22) Date of filing: 31.05.1995
(51) Int. Cl.: A61K 39/02, A61L 2/08, A61L 2/16

(54) **TREATMENT OF PLASMA**
BEHANDLUNG VON BLUT-PLASMA
TRAITEMENT DU PLASMA SANGUIN

(30) Priority: 01.06.1994 FI 942576
(43) Date of publication of application: 19.03.1997
(73) Proprietor: SUOMEN PUNAINEN RISTI VERIPALVELU, 00310 Helsinki (FI)
(72) Inventor: PARKKINEN, Jaakko, 02150 Espoo (FI)
(74) Representative: Heikkinen, Esko Juhani
(86) International application number: FI9500306
(87) International publication number: WO9532732

(56) References cited:
- WO-A-93/00005
- US-A- 4 720 385
- US-A- 4 878 891

## Description

### Field of the invention

This invention relates to the treatment of plasma and plasma products during photochemical virus inactivation.

### Background of the Invention

Transfusion of blood and blood products carries a certain risk of transmission of infectious diseases. The risk can be much reduced by proper blood donor selection and by testing the donors for infection markers. However, the existence of a "window period" in the appearance of infection markers makes it impossible to detect current viral infections, such as viral hepatitis and HIV-infection. This is an especially important consideration when plasma is collected from high-risk populations.

Several different virus inactivation methods are currently used for blood products. Depending on the principle of the inactivation method, it may be used only for certain types of blood products. For example, heating and solvent/detergent treatment can be used for virus inactivation of plasma and purified plasma proteins but not for blood cells.

Photochemical treatment (also called photodynamic treatment, photooxidation or photosensitization) has been used to inactivate viruses in whole blood, red cell and platelet concentrates, and fresh frozen plasma (e.g., Matthews JL, Newman JT, Sogandares-Bernal F, et al., Photodynamic therapy of viral contaminants with potential for blood banking applications, Transfusion 1988, 28:81-3). The principle of the photochemical treatment is that a photoactive compound or photosensitizer, when excited with irradiation, carries out secondary reactions that inactivate viruses. Photoactive substances used with blood components include porphyrins, phenothiazine dyes, cyanine derivatives, and psoralens. The wavelength of irradiation depends on the excitation spectrum of the photoactive compound used.

A photochemical treatment employing phenothiazine dyes, especially methylene blue (MB) as photosensitizer has been described for the virus inactivation of fresh frozen plasma (FFP) (see Mohr H, Lambrecht B. Verfahren zur Inaktivierung von Vieren in Blut und Blutprodukten, Offenlegungsschrift DE 3930510 A1). Methylene blue has its absorption maximum at 660 nm. In the described method illumination is carried out with fluorescent tubes which emit most of their radiation energy below 600 nm. Recently, light emitting diodes (LEDs) with a sharp emission maximum at 660 nm were reported to be more effective than the fluorescent tubes in the MB-mediated virus inactivation in plasma (Lambrecht B, Mohr, H, Schmitt H, Influence of light quality and quantity on the photodynamic inactivation of viruses in fresh frozen plasma in the presence of methylene blue, International Society of Blood Transfusion, IV Regional Congress, European Region, Barcelona 1993, p. 329).

There are several possible reactions that may take place after excitation of the photoactive compound. They can be classified into three main groups: Reactions due to radicals, reactions due to singlet oxygen, and those that do not involve oxygen. When using MB, the main mediator of photooxidation in plasma is probably singlet oxygen. However, binding of MB to macromolecules, especially viral nucleic acid, may also promote direct electron transfer reactions (OhUigin C, McConnell DJ, Kelly JM, van der Putten WJM, Methylene blue photosensitized strand cleavage of DNA: effects of dye binding and oxygen, Nucleic Acid Res. 1987, 15:7411-27). The photochemical treatment inactivates with a few exceptions only enveloped viruses. This may be due to the higher solubility of MB and oxygen in the lipid membrane of the enveloped viruses than in the aqueous phase of plasma. The exact mechanism of virus inactivation by MB treatment is not known but it may depend on oxidation of the viral nucleic acid, capsid proteins or the viral membrane that mediates the binding and entry of the virus into the host cell.

Besides viral structures, the photochemical treatment may also result to oxidation of endogenous plasma components, i.e., plasma proteins, lipoprotein particles and small molecular weight compounds. This leads e.g. to decrease in coagulation factor activities which appears to be up to 30 %. This is clearly not desirable since plasma is transfused especially to replenish coagulation factors. At present, there is insufficient information on the possible individual variation between different plasma units in their capacity to resist inactivation of plasma proteins during photooxidation. This is an important consideration since plasma is treated with MB as single donor plasma units.

According to our knowledge, there are no published data on attempts to protect plasma proteins against photooxidation. There are, however, a few reports on the effect of antioxidants on photooxidation of blood cells. E.g. it has been reported that ascorbate (10-100 *µ*mol/l) stimulated photohemolysis (Sorata Y, Takahama U, Kimura M, Cooperation of quercetin with ascorbate in the protection of photosensitized lysis of human erythrocytes in the presence of hematoporphyrin, Photochem Photobiol 1988, 48:195-9). Also the importance of endogenous antioxidants in plasma against oxidation of lipoproteins has been studied. Ascorbate has been found to protect plasma lipids effectively against aqueous peroxyl radicals (e.g. Frei B, Stocker R, Ames BN, Antioxidant defenses and lipid peroxidation in human blood plasma, Proc Natl Acad Sci USA, 1988, 85:9748-52). In contrast it has been concluded that oxidative damage to plasma proteins is not effectively prevented by any of the plasma antioxidants (Frei B, England L, Ames BN, Ascorbate is an outstanding antioxidant in human blood plasma, Proc Natl Acad Sci USA, 1989, 86:6377-81). When singlet oxygen generating endoperoxides were studied, it was estimated that most of singlet oxygen generated in the aqueous phase of plasma was quenched by plasma proteins (63 %), urate (9 %) and bilirubin (5 %) (Wagner JR, Motchnik PA, Stocker R, Sies H, Ames BN, The Oxidation of blood plasma and low density lipoprotein components by chemically generated singlet oxygen, J Biol Chem, 1993, 268:18502-6). Ascorbate did not contribute significantly to singlet oxygen quenching (0.6 %).

### Summary of the Invention

Our results show that the decrease in the functional activity of plasma constituents, especially coagulation factors, during photochemical treatment is dependent on the concentration of ascorbate in the plasma perparation. By adding exogenous ascorbate to the preparation, coagulation factors can be effectively protected from inactivation. Effective protection of coagulation factors is achieved when the initial ascorbate concentration is 100-300 µmol/l which should be reached in most plasma units by adding 100-150 µmol/l of ascorbate. The upper limit of the concentration is 1000 µmol/l because at high concentrations ascorbate may reduce the photoactive substance. At least, initial ascorbate level should be higher than the utilization of ascorbate during the treatment which was in our experiments about 100 µmol/l in plasma packs. The utilization of ascorbate can be used as an accurate measure of the intensity of the photochemical treatment conditions. Ascorbate should protect plasma proteins also if the photochemical treatment would be carried out for virus inactivation of purified plasma proteins or fractions instead of whole plasma.

Ascorbate can be added as ascorbic acid or as any suitable derivative thereof. Ascorbate can be added either before, simultaneously, or after the addition of photoactive substance to plasma. Ascorbate and photoactive substance can be added also in the same solution containing, e.g. 50-500-fold, molar excess of ascorbate.

Suitable photoactive substances include e.g. phenothiazine dyes (especially methylene blue), porphyrins, and cyannine derivatives, which are excited by visible light, and psoralens, which are excited be UV light.

The method can be used especially for the treatment of fresh plasma or fresh frozen plasma, but it is also suitable for the treatment of other protein preparations derived from plasma.

The invention and some of its preferable embodiments are defined in detail in the claims.

### Brief Description of Drawings

The enclosed drawings relate to the experiments described herein.
- Fig. 1 shows the effect of plasma ascorbate on inactivation of fibrinogen and F VIII during photodynamic treatment.
- Fig. 2 shows the effect of plasma ascorbate on vesicular stomatitis virus inactivation.
- Fig. 3 shows the recovery of clottable fibrinogen in 12 FFP units after photodynamic treatment.
- Fig. 4 shows the recovery of F VIII activity in 12 FFP units after photodynamic treatment.
- Fig. 5 shows the disappearance of ascorbate from plasma during photodynamic treatment.
- Fig. 6 shows the utilization of ascorbate during the photodynamic treatment of plasma packs.
- Fig. 7 shows the recovery of clottable fibrinogen in cryoprecipitate prepared from MB-treated plasma.
- Fig. 8 shows the recovery of F VIII activity in cryoprecipitate prepared from MB-treated plasma

### Experiments

To study the effect of possible protecting compounds against photooxidation, we illuminated 2 ml samples of pooled plasma containing 1 *µ*mol/l of MB and the compounds studied with the TL-M 115/33 fluorescent tubes for 1 h. These conditions are similar to those described for the MB treatment of FFP units (Mohr H, Pohl U, Lambrecht B, Wieding JU, Schmitt H, Durch Methylenblau/Lichtbehandlung virusinaktiviertes Humanplasma: Herstellung und bisherige klinische Erfahrungen, Infusionsther Transfusionsmed 1993, 20:19-24). Twelve different coagulation factors were measured before and after the treatment. The extent of inactivation was largest for fibrinogen and factor VIII, about 50 and 40 %, respectively. Of the different compounds tested, ascorbate was found to protect most effectively all coagulation factors studied against photooxidation. The protecting effect was dose-dependent at concentrations of 50-600 *µ*mol/l (Example 1). The plasma pool used for these experiments had endogenous ascorbate level of 35 *µ*mol/l which is clearly less than the average level of blood donors (about 60 *µ*mol/l). When ascorbate concentration was increased from 35 to 135 *µ*mol/l, the inactivation of F VIII and fibrinogen decreased by 60 % indicating a steep dose-response effect at these ascorbate concentrations. At higher ascorbate concentrations, the increase in the protecting effect was more gradual and approached complete protection at concentrations over 600 *µ*mol/l. These ascorbate concentrations had only a slight retarding effect on the inactivation of vesicular stomatitis virus (VSV) during the photochemical treatment (Example 1).

Plasma ascorbate levels may vary between a few µmol/l to more than 100 *µ*mol/l. In a population of 156 Finnish men, the mean was 46 and the range 4-125 *µ*mol/l. To study the significance of endogeneous plasma ascorbate during photochemical treatment of individual plasma units, we treated 12 FFP units with MB and plotted the residual activity of coagulation factors after the treatment against the initial plasma ascorbate level (Example 2). The residual activity of fibrinogen and F VIII correlated directly to the initial plasma ascorbate level. This further emphasized the importance of plasma ascorbate in resisting protein inactivation during the photochemical treatment of single donor plasma units.

To study the fate of ascorbate during the photochemical treatment, we followed the concentration of ascorbate at different stages of the treatment and compared it to urate, another antioxidant considered important in plasma. It was found that ascorbate decreased rapidly from plasma. When plasma was illuminated in the plastic tubes, the decrease was 100 *µ*mol/l during 45 min photooxidation (Example 3). Urate also disappeared from plasma but clearly more slowly than ascorbate. Furthermore, the rate of urate utilization was dependent on the initial level of ascorbate suggesting that ascorbate protected urate during MB treatment. This emphasized the primary role of ascorbate as a physiological antioxidant in plasma against photooxidation. Ascorbate disappeared from plasma slower when the illumination was carried out in plasma packs than in the plastic tubes (100 *µ*mol/60 min vs. 100 *µ*mol/45 min). Since the extent of coagulation factor inactivation was also larger in the plastic tubes than in the plasma packs, this suggests that the rate of ascorbate utilization can be used as an accurate measure of the intensity of the photochemical treatment.

Since MB-treated plasma might provide a source for the preparation of virus-inactivated cryoprecipitate for the treatment of various bleeding disorders, we studied also the influence of plasma ascorbate on coagulation factor activities in cryoprecipitates prepared from MB-treated plasma. For this purpose, we subjected three plasma pools to MB treatment in plasma packs with different levels of ascorbate. Four units of 200 IU cryoprecipitate were prepared from each plasma pool (Example 4). Supplementation of 50 to 100 *µ*mol/l of ascorbate to the plasma before MB treatment was found to clearly increase the amount of clottable fibrinogen and F VIII activity in the cryoprecipitate.

To find out whether ascorbate and MB could be added simultaneously to plasma, we studied the stability of concentrated solutions of ascorbate and MB. We found that when a concentrated solution of ascorbate and MB was prepared which contained 30 *µ*mol/l of MB and 3 mmol/l of ascorbate at pH 3, MB was reduced to a nonabsorbing form. However, after dilution of the mixture to a neutral buffer or to plasma, the absorbing form of MB was quantitatively restored. This type of solutions of MB and ascorbate were stable for several days in dark, which provides a convenient way for the addition of ascorbate and MB simultaneously to plasma packs.

### Example 1. Effect of initial plasma ascorbate concentration on coagulation factor and virus inactivation during MB treatment of plasma.

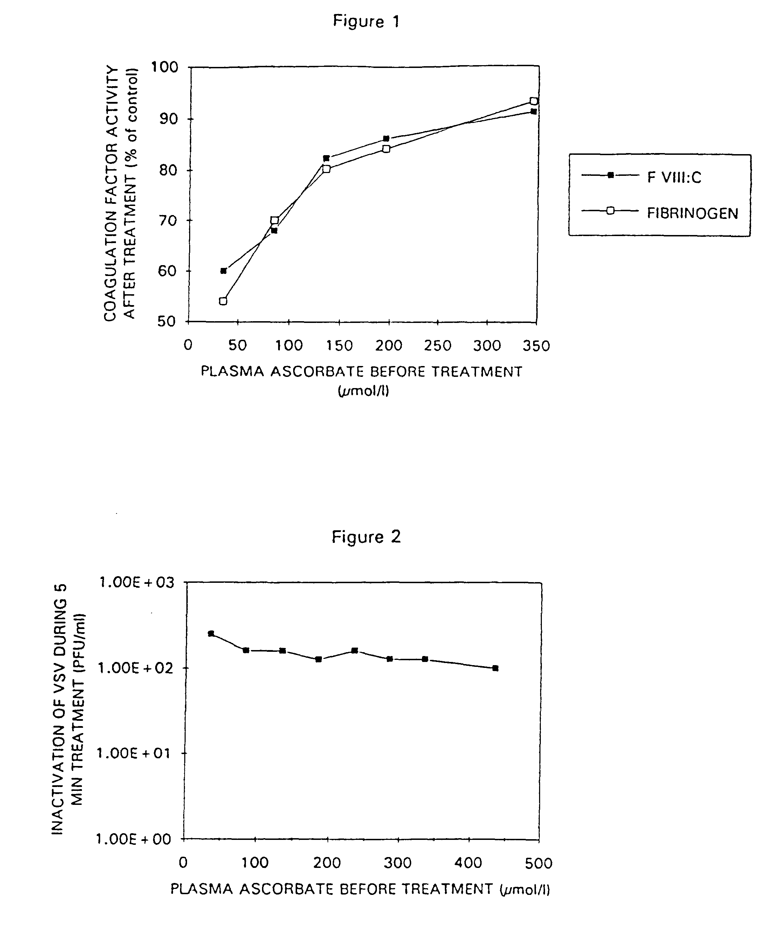

To study the effect of added ascorbate, we used a plasma pool with a low ascorbate level (35 *µ*mol/l). Ascorbate was measured by high-pressure liquid chromatography as described before (Parviainen MT, Nyyssönen K, Penttilä IM, Seppänen K, Rauramaa R, Salonen JTS, Gref C-G, A method for routine assay of plasma ascorbic acid using high-performance liquid chromatography, J Liquid Chrom 1986, 9:2185-97). Ascorbate was added to the plasma at different concentrations before MB that was used at a final concentration of 1 *µ*mol/l. Plasma samples of 2 ml were illuminated in 2.5 ml cryotubes (Falcon) with TL-M 115/33 RS fluorescent tubes (Philips) for 1 h. A transparent cooling plate with fluid circulation was placed between the plasma samples and the fluorescent tubes to keep the temperature of the plasma below 22 °C. The irradiation power at the level of the plasma tubes was 1.9 mW/cm² at 595-695 nm (40 000 lux). After illumination, the samples were divided into portions of 0.5 ml and frozen. F VIII activity was measured with a chromogenic substrate (Coatest Factor VIII, Chromogenix) and clottable fibrinogen with a thromboplastin time assay (Thromboplastin, International Laboratories) (Rossi E, Mondonico P, Lombardi A, Preda L, Method for the determination of functional (clottable) fibrinogen by the new family ACL coagulometers, Thromb Res 1988, 52:453-6818). The inactivation of all coagulation factors studied (F I, F II, F VII, F VIII, F IX, F X, protein C) was found to be reduced when increasing concentrations of ascorbate were added to the plasma samples. The dose-response curves are shown for fibrinogen and F VIII in Fig. 1.

For virus inactivation studies, 5 % (by vol.) of vesicular stomatitis virus (VSV) culture supernatant was added to the plasma before ascorbate and MB. The virus titer in the plasma samples was determined with plaque titration before and after illumination (the titer was typically 10⁴/ml before illumination). The inactivation of VSV during the MB treatment was only slightly retarded by the increasing concentrations of ascorbate (Fig. 2). Virus inactivation was determined after 5 min treatment when the virus titer was still over 10²/ml because this allowed more accurate plaque titration.

### Example 2. Effect of endogeneous plasma ascorbate levels on coagulation factor inactivation during MB treatment of plasma.

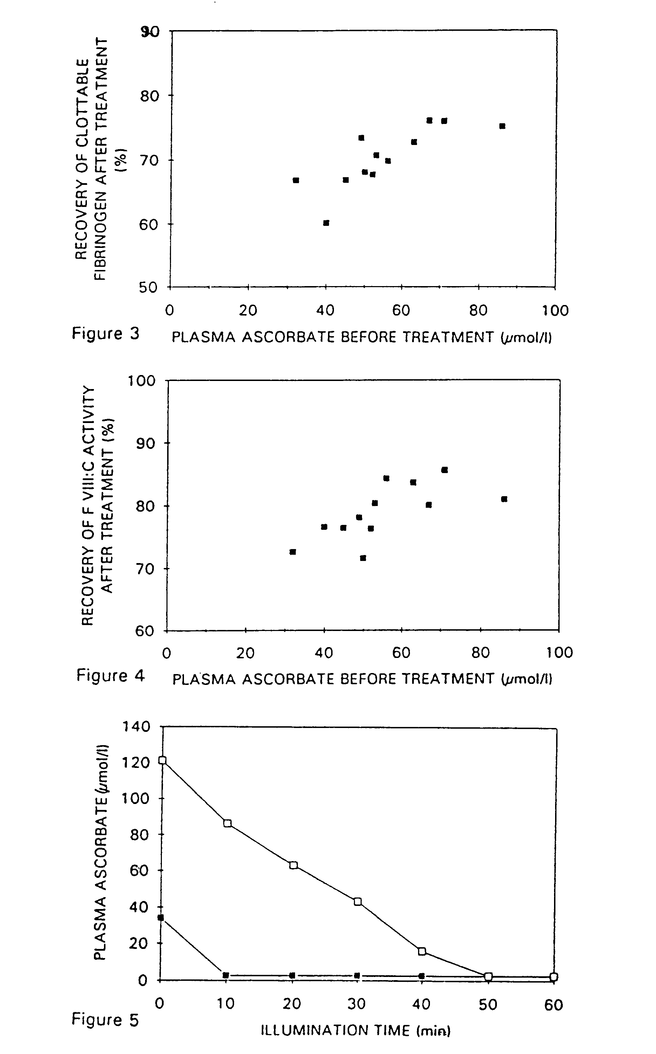

Since added ascorbate had a clear protecting effect, we studied the significance of endogeneous plasma ascorbate level on the inactivation of coagulation factors during the MB treatment. Fresh frozen plasma units from 12 blood donors were treated with 1 *µ*mol/l of MB and illumination with the fluorescent tubes for 1 h. Samples were taken from the plasma units before and after MB treatment for coagulation factor and ascorbate determinations. Fibrinogen was determined with the a Clauss method (Fibrinogen-C, International Laboratories). This method was more sensitive than the thromboplasmin time assay for changes in fibrinogen caused by the MB treatment. The preservation of the coagulation factor activities during the MB treatment showed direct correlation to the initial plasma ascorbate level of the FFP units (Figs. 3 and 4).

### Example 3. Effect of MB treatment on plasma ascorbate and urate.

To study the fate of ascorbate during MB treatment, we followed the concentration of ascorbate at different stages of the treatment and compared it to urate, another antioxidant considered important in plasma. Plasma was illuminated with the fluorescent tubes in 2 ml portions as in Example 1, and the illumination was interrupted at different periods. Urate was measured with the same HPLC method as ascorbate. It was found that ascorbate disappeared steadily from plasma. When plasma was illuminated in the plastic tubes with the fluorescent tubes, the decrease was 100 *µ*mol/l during a 45 min photooxidation (Fig. 5). Urate also disappeared from plasma but clearly more slowly than ascorbate. Furthermore, a high initial level of ascorbate protected urate from oxidation indicating that ascorbate is primary to urate as an antioxidant during MB treatment.

### Example 4. Effect of plasma ascorbate level on coagulation factor activity in cryoprecipitates prepared from MB-treated plasma.

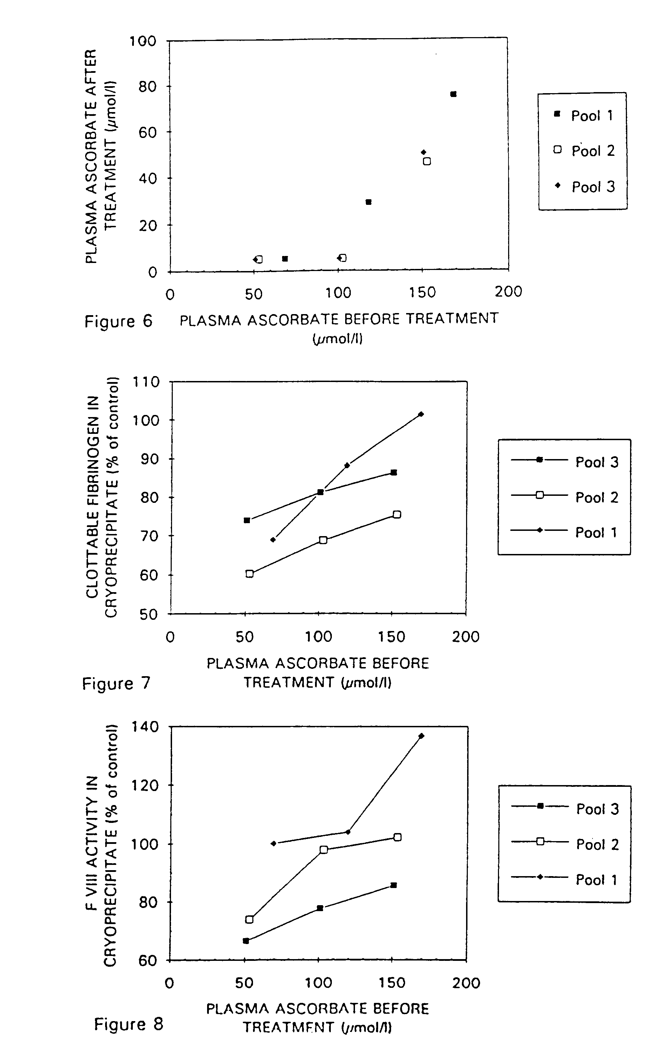

We next looked how the initial plasma ascorbate level influences the quality of cryoprecipitate prepared from MB-treated plasma. Three 2 l plasma pools were made and each was divided into eight 250 ml plasma packs. Two packs were kept as a control and the remaining packs were subjected to MB treatment. Two packs were treated under identical conditions either without ascorbate addition or after addition of 50 or 100 *µ*mol/l ascorbate. MB treatment was carried out with 1 *µ*mol/l MB and illumination for 30 min with a LED illumination device (21 mW/cm² at 595-695 nm). Under these conditions, 100 *µ*mol/l of ascorbate disappeared from plasma during the illumination (Fig. 6).

After MB treatment, two identically treated packs were combined and the resulting 500 ml of plasma was frozen for preparation of cryoprecipitate ("200 IU cryoprecipitate"). Cryoprecipitates were dissolved into 25 ml of buffer and samples were frozen for coagulation factor determinations. F VIII activity was determined with a one-stage APTT method and clottable fibrinogen with the Clauss method. The results for the MB-treated cryoprecipitates are shown as percentage of the nontreated cryoprecipitate. As shown in Figs. 7 and 8, fibrinogen and F VIII activities in the dissolved cryoprecipitates correlated directly to the initial level of ascorbate in the MB-treated plasma packs.

## Claims

1. A method for the treatment of a blood plasma preparation in order to inactivate possible viruses in the preparation, comprising adding ascorbate to the preparation, wherein the amount of the added ascorbate is such that the concentration of the ascorbate after the addition is 100-1000 µmol/l, adding a photoactive substance to the preparation, and irradiating the preparation at a wavelength which excites the photoactive substance.

2. A method in accordance with claim 1, wherein the plasma preparation is fresh plasma or fresh frozen plasma.

3. A method in accordance with claim 1 or 2, wherein phenothiazine dyes, porphyrins, cyanine derivatives, or psoralens are used as the photoactive substance.

4. A method in accordance with claim 3, wherein phenothiazine dyes are used as the photoactive substance.

5. A method in accordance with claim 1 or 2, wherein the plasma preparation is irradiated by visible light.

6. A method in accordance with any of claims 1 to 5, wherein the amount of the added ascorbate is such that the concentration of the ascorbate after the addition is 100-300 µmol/l.

7. A method in accordance with any of claims 1 to 5, wherein the amount of the added ascorbate is at least 100 µmol/l.

8. A method in accordance with any of claims 1 to 5, wherein the mole amount of the added ascorbate is about 50-500 times the mole amount of the added photoactive substance.

## Patentansprüche

1. Verfahren zur Behandlung eines Blutplasma-Präparats zur Inaktivierung möglicher Viren im Präparat, umfassend die Zugabe von Ascorbat zum Präparat, wobei das Ascorbat in solcher Menge zugesetzt wird, dass die Konzentration des Ascorbats nach der Zugabe 100-1000 µmol/l beträgt, die Zugabe einer fotoaktiven Substanz zum Präparat, und die Bestrahlung des Präparats mit einer Wellenlänge, die die fotoaktive Substanz anregt.

2. Verfahren nach Anspruch 1, wobei das Plasma-Präparat frisches Plasma oder frisches gefrorenes Plasma ist.

3. Verfahren nach Anspruch 1 oder 2, wobei Phenothiazin-Farbstoffe, Porphyrine, Cyaninderivate oder Psoralene als fotoaktive Substanzen verwendet werden.

4. Verfahren nach Anspruch 3, wobei Phenotiazin-Farbstoffe als fotoaktive Substanz verwendet werden.

5. Verfahren nach Anspruch 1 oder 2, wobei das Plasma-Präparat mit sichtbarem Licht bestrahlt wird.

6. Verfahren nach einem der Ansprüche 1 bis 5, wobei das Ascorbat ins solcher Menge zugesetzt wird, das die Konzentration des Acorbats nach der Zugabe 100-300 µmol/l beträgt.

7. Verfahren nach einem der Ansprüche 1 bis 5, wobei die Menge des zugesetzten Ascorbats mindestens 100 µmol/l beträgt.

8. Verfahren nach einem der Ansprüche 1 bis 5, wobei die Mol-Menge des zugesetzten Ascorbats etwa das 50-500 fache der Mol-Menge der zugesetzten fotoaktiven Substanz beträgt.

## Revendications

1. Méthode de traitement d'une préparation de plasma sanguin pour inactiver les éventuels virus se trouvant dans la préparation, consistant à additionner de l'ascorbate à la préparation, la quantité d'ascorbate additionnée étant telle que la concentration d'ascorbate après addition est comprise entre 100 et 1000 *µ*mol/l, à additionner une substance photoactive à la préparation, et à irradier la préparation à une longueur d'onde qui excite la substance photoactive.

2. Méthode selon la revendication 1, **caractérisée en ce que** la préparation de plasma est du plasma sanguin frais ou du plasma sanguin frais congelé.

3. Méthode selon la revendication 1 ou 2, **caractérisée en ce que** l'on utilise comme substance photoactive des colorants de phénothiazine, des porphyrines, des dérivés de cyanine, ou des psoralènes.

4. Méthode selon la revendication 3, **caractérisée en ce que** l'on utilise comme substance photoactive des colorants de phénothiazine.

5. Méthode selon la revendication 1 ou 2, **caractérisée en ce que** la préparation de plasma est irradiée par la lumière du visible.

6. Méthode selon l'une quelconque des revendications 1 à 5, **caractérisée en ce que** la quantité d'ascorbate additionné est telle que la concentration d'ascorbate après addition est comprise entre 100 et 300 *µ*mol/l.

7. Méthode selon l'une quelconque des revendications 1 à 5, **caractérisée en ce que** la concentration d'ascorbate additionné est au moins de 100 *µ*mol/l.

8. Méthode selon l'une quelconque des revendications 1 à 5, **caractérisée en ce que** la quantité molaire d'ascorbate additionné est environ 50 à 500 fois plus importante que la quantité molaire de substance photoactive additionnée.
